# EUROPEAN PATENT SPECIFICATION

(11) **EP 2 040 689 B1**
(45) Date of publication and mention of the grant of the patent: **01.12.2010**
(21) Application number: 07764759.2
(22) Date of filing: 21.06.2007
(51) Int. Cl.: A61K 31/191, A61K 31/198, A61K 31/375, A61K 38/06, A61K 38/38, A61K 45/06, A61K 9/08

(54) **SOLUTION COMPRISING N-ACETYLCYSTEINE AND GLUCONIC ACID, AND METHODS TO REDUCE, TREAT AND/OR PREVENT OXIDATIVE STRESS AND CELL ACTIVATION**
LÖSUNG ENTHALTEND N-ACETYLCYSTEIN UND GLUCONSÄURE, UND VERFAHREN ZUR VERRINGERUNG, BEHANDLUNG UND/ODER VERHINDERUNG VON OXIDATIVEM STRESS UND ZELLAKTIVIERUNG
SOLUTION CONTENANT DE LA N-ACÉTYLCYSTÉINE ET DE L'ACIDE GLUCONIQUE, ET MÉTHODE RÉDUISANT, TRAITANT ET/OU PRÉVENANT LE STRESS OXYDATIF ET L'ACTIVATION CELLULAIRE

(30) Priority: 22.06.2006 US 805489 P
(43) Date of publication of application: 01.04.2009
(73) Proprietor: Gambro Lundia AB, 22 643 Lund (SE)
(72) Inventor: DEPPISCH, Reinhold, 72379 Hechingen (DE); BECK, Werner, 72108 Rottenburg (DE); SCHNELL, Andrea, 72406 Bisingen-Thanheim (DE); DRIESCHMANNS, Hans-Rainer, 72072 Tübingen (DE)
(74) Representative: Hornung, Veronika Margot
(86) International application number: PCT/EP2007/005466
(87) International publication number: WO 2007/147590

(56) References cited:
- EP-A- 0 158 487
- WO-A-00/64421
- WO-A-01/02004
- WO-A-01/28544
- WO-A-2007/046757
- JP-A- 8 116 993
- US-A1- 2002 068 268
- DE BACKER W A ET AL: "N-acetylcysteine pretreatment of cardiac surgery patients influences plasma neutrophil elastase and neutrophil influx in bronchoalveolar lavage fluid" INTENSIVE CARE MEDICINE, vol. 22, no. 9, 1996, pages 900-908, XP009093435 ISSN: 0342-4642
- SCHOLZE A ET AL: "Acetylcysteine reduces plasma homocysteine concentration and improves pulse pressure and endothelial function in patients with end-stage renal failure." CIRCULATION 27 JAN 2004, vol. 109, no. 3, 27 January 2004 (2004-01-27), pages 369-374, XP007903732 ISSN: 1524-4539
- DAVIDE SOLDINI ET AL: "Pharmacokinetics of N-acetylcysteine following repeated intravenous infusion in haemodialysed patients" EUROPEAN JOURNAL OF CLINICAL PHARMACOLOGY, vol. 60, no. 12, 1 February 2005 (2005-02-01), pages 859-864, XP019329945 ISSN: 1432-1041
- LUND M E ET AL: "TREATMENT OF ACUTE METHYLMERCURY INGESTION BY HEMODIALYSIS WITH N-ACETYLCYSTEINE (MUCOMYST) INFUSION AND 2,3-DIMERCAPTOPROPANE SULFONATE" JOURNAL OF TOXICOLOGY. CLINICAL TOXICOLOGY, MARCEL DEKKER, NEW YORK, NY, US, vol. 22, no. 1, July 1984 (1984-07), pages 31-49, XP000991147 ISSN: 0731-3810
- LOPEZ-GONZALEZ M A ET AL: "Ototoxicity caused by cisplatin is ameliorated by melatonin and other antioxidants" JOURNAL OF PINEAL RESEARCH, vol. 28, no. 2, March 2000 (2000-03), pages 73-80, XP002203801 ISSN: 0742-3098
- ESREFOGLU M. ET AL: "Antioxidative effect of melatonin, ascorbic acid and N-acetylcysteine on caerulein-induced pancreatitis and associated liver injury in rats." WORLD JOURNAL OF GASTROENTEROLOGY, vol. 12, no. 2, 14 January 2006 (2006-01-14), pages 259-264, XP007903651 ISSN: 1007-9327
- ORTOLANI O ET AL: "The effect of glutathione and N-acetylcysteine on lipoperoxidative damage in patients with early septic shock." AMERICAN JOURNAL OF RESPIRATORY AND CRITICAL CARE MEDICINE, vol. 161, no. 6, June 2000 (2000-06), pages 1907-1911, XP007903642 ISSN: 1073-449X
- DATABASE WPI Week 200639 Derwent Publications Ltd., London, GB; AN 2006-376218 XP002461240 & JP 2006 137745 A (NISSHO KK) 1 June 2006 (2006-06-01)
- DRIESCHMANNS H -R ET AL: "Inhibition of PMA-induced reactive oxygen species (ROS) by antioxidants" INTERNATIONAL JOURNAL OF ARTIFICIAL ORGANS, vol. 29, no. 5, May 2006 (2006-05), page 518, XP007903322 & 32ND CONGRESS OF THE EUROPEAN-SOCIETY-OF-ARTIFICIAL-ORGANS; UMEA, SWEDEN; JUNE 21 -24, 2006 ISSN: 0391-3988
- DONCHEVA ET AL: "PO22-706 OXIDATIVE STRESS MARKERS IN HEMODIALYSIS PATIENTS TREATED BY A NOVEL ANTIOXIDANT CONTAINING DIALYSIS SOLUTION" ATHEROSCLEROSIS SUPPLEMENTS & 76TH CONGRESS OF THE EUROPEAN ATHEROSCLEROSIS SOCIETY, 10-13 JUNE 2007, HELSINKI, FINLAND, vol. 8, no. 1, June 2007 (2007-06), page 189, XP022151086 ISSN: 1567-5688

## Description

### TECHNICAL FIELD

The present invention concerns a solution to reduce, treat and/or prevent oxidative stress and cell activation.

### BACKGROUND OF THE INVENTION

Cell activation and circulation of activated cells, which leads to or initiates severe tissue destruction, is a generalized problem in many acute or chronic diseases, i.e. acute or chronic renal failure, liver failure, post surgical trauma, bums, peritoneal dialysis, as well as in ex vivo or in vitro in cell culture systems. Cell activation is defined as induction of pro-inflammatory gene expression, induction of cytokine production and release, enhanced expression of cell surface markers which modulate invasion or adherence of circulating immune cells to the vascular wall.

One important consequence of cell activation is the generation of a milieu of enhanced oxidative stress. The formation of reactive oxygen species (ROS) both at the intra- and extra-cellular level is a major component of host defence mechanism in human being. Oxidative balance is an integral component of the internal milieu homeostasis resulting from a perfect balance between oxidative and anti-oxidative mechanism. An imbalance of this system is called oxidative stress and results both from severely impaired (e.g. depleted) oxygen radical scavenger system and enhanced reactive oxygen species production.

Oxidative stress may enhance cell activation by autocrine, paracrine and indirectly also by endocrine action of reactive oxygen species, thereby perpetuating different cell activation signals, such as nuclear transcription of proinflammatory genes (e.g. NF□B-related), synthesis and release of cytokines (e.g. IL-6) and chemokines and expression of cell surface receptors (e.g. CD11b). In other words: there is a primary stimulus leading to oxidative stress and if oxidative stress persists cell activation is enhanced.

Autocrine action means than a cell secretes a chemical compound (e. g. reactive oxygen species) and this compound causes other signals the same cell.

Paracrine action means that the target cell is close to the signal releasing cell, and the signal chemical is broken down too quickly to be carried to other parts of the body.

Indirect endocrine action means that as a consequence of the autorcine or paracrine action of short-living reactive oxygen species longer living signal molecules (e.g. hormones, cytokines or reaction products of reactive oxygen species and proteins or other biological molecules) are released into the blood stream and act on target cells distributed throughout the body. Endocrine signalling is usually - but not exclusively -chronic, in other words, acts in the long term.

An intervention to treat and/or prevent cell activation and/or oxidative stress means that different compounds which are responsible for the cell activation signalling are regulated via scavenging of reactive oxygen species at different levels, e.g. cytokine induction and release, surface marker expression and nuclear transcription of proinflammatory genes. This may include indirect action in a sense that a compound which has no direct scavenging effect on reactive oxygen species may act by enhancing the synthesis or bioavailability of one or more other compounds which have such a scavenging effect.

This may also include the combination of different compounds comprising directly acting, i.e. scavenging antioxidative substances and indirectly acting substances. ,

Several attempts have been made to address this problem. WO 2001/28544 describes the use of free radical scavengers within dialysis solutions for gamma sterilisation.

US 5,474,992 discloses a specific antioxidant in a dialysis solution or for injection/infusion.

WO 93/14796 discloses a PD solution containing an agent to scavenge free radicals, such as vitamin E, procystein or superoxide dismuutase.

WO 2004/014355 describes a PD solution with pyruvate and one additional antioxidant.

WO 2001/02004 refers to a PD solution with one or more antioxidants, such as N-acetyl-cysteine, which are added to inhibit reactive oxygen species.

WO 2000/64421 describes a method of decreasing the effect of oxidative stress in a patient having renal disease and undergoing chronic hemodialysis, comprising administering intravenously N-acetyl-cysteine.

US 6,355,682 discloses a method for treating damages caused by acute renal failure, said method comprising administering N-acetyl-cysteine.

WO 99/07419 relates to a method of treating nutrient deficiencies in hemodialysis and PD patients. Patients are dialyszed with a solution containing at least one vitamin.

US 2003/0206972 is directed to a formulation which is designed to inhibit free radical formation and oxidative stress in warm blooded animals.

Further, several other publications deal with this problem.

Witko-Sarsat et al. discuss AOPP-induced activation of human neutrophil and monocyte oxidative metabolism as being a potential target for N-acetylcysteine treatment in dialysis patients (Kidney Int 2003 Jul;64:82-91).

Alonso et al. refer to the prevention of radiocontrast nephropathy with N-acetylcysteine in patients with chronic kidney disease and trials related thereto (Am J Kidney Dis 2004 Jan;43:1-9).

Zaniew et al. discuss the influence of vitamin E and N-acetylcysteine on intracellular oxidative stress in T lymphocytes in children treated with dialysis (Wiad Lek 2005;58 Suppl 1:58-65).

Ortolani et al. investigate the effect of glutathione and N-acetylcysteine on lipoperoxidative damage in patients with early septic shock (Am J Respir Crit Care Med 2000 Jun; 161:1907-11).

Heller et al. describe that N-acetylcysteine reduces respiratory burst but augments neutrophil phagocytosis in intensive care unit patients (Crit Care Med 2001 Feb;29:272-6).

Shimizu et al. published an article titled "N-acetylcysteine attenuates the progression of chronic renal failure" in Kidney Int 2005 Nov;68:2208-17.

Soldini et al. deal with the pharmacokinetics of N-acetylcysteine following repeated intravenous infusion in haemodialysed patients (Eur J Clin Pharmacol 2005 Feb;60:859-64).

Allegra et all refer to human neutrophil oxidative bursts and their in vitro modulation by different N-acetylcysteine concentrations (Arzneimittelforschung 2002;52:669-76).

Friedman et al. discuss the effect of N-acetylcysteine on plasma total homocysteine levels in hemodialysis in a randomized, controlled study (Am J Kidney Dis 2003 Feb;41:442-6).

Tepel et al. discuss the ability of the antioxidant acetylcysteine to reduce cardiovascular events in patients with end-stage renal failure (Circulation. 2003 Feb 25;107(7):992-5).

Accordingly, cell activation and oxidative stress is recognised as a general metabolic disturbance leading to severe tissue destruction and malfunction of organs, for example kidney and liver and also including destruction of the vascular system.

### SUMMARY OF THE INVENTION

One object of the present invention is to reduce, treat and/or prevent oxidative stress and/or cell activation e.g. in an extracorporeal setting (for blood treatment or in ex vivo cell culturing and handling/delivery systems) through the membrane by adding a solution according to the invention to the dialysis fluid, or by infusion of a solution according to the invention.

The present invention relates to a solution to reduce, treat and/or prevent oxidative stress and cell activation. According to the invention the solution gives rise to a concentration within a blood flow of 0.1-18 mM N-acetyl-cysteine, preferably 0.1-10 mM N-acetyl-cysteine and gives rise to a gluconic acid concentration within a blood flow of 0.4-26 mM, preferably 2-26 mM gluconic acid, and more preferably 2-12 mM.

It may further be desirable to add to said N-acetyl-cysteine and gluconic acid comprising solution, vitamin C in a concentration which gives rise to a concentration within a blood flow of 0.01-0.21 mM vitamin C, preferably 0.05-0.2 mM.

It may further be desirable to add to the above-mentioned solutions comprising N-acetyl-cysteine and gluconic acid, or comprising N-acetyl-cysteine, vitamin C and gluconic acid, glutathione in a concentration which gives rise to a concentration in the blood flow of 0.01-5 mM glutathione, preferably 0.01-0.5 mM.

In an embodiment of the invention the solution gives rise to the following concentrations of constituents within a blood flow of 0.1-18 mM, preferably 0.1-10 mM N-acetyl-cysteine, 0.01-0.21 mM, preferably 0.05-0.2 mM vitamin C and 2-26 mM, preferably 2-12 mM gluconic acid.

In another embodiment of the invention the solution gives rise to the following concentrations of constituents within a blood flow of 0.1-18 mM, preferably 0.1-10 mM N-acetyl-cysteine, 0.01-0.21 mM, preferably 0.05-0.2 mM vitamin C, 0.4-26 mM, preferably 2-26 mM, and more preferably 2-12 mM gluconic acid and 0.01-5 mM, preferably 0.01-0.5 mM glutathione.

In yet another embodiment of the invention a solution according to the invention adds an additional amount of 0.2 - 2 weight-% human serum albumin to the blood flow.

Therefore, in another embodiment of the invention, anyone of the solutions described above, each further comprises 0.2-20 weight-% human serum albumin.

One method to reduce, treat and/or prevent oxidative stress and cell activation comprises infusing a solution according to anyone of the embodiments above into the blood circuit of a patient in need thereof.

Another method to reduce, treat and/or prevent oxidative stress and cell activation comprises infusing a solution according to anyone of the embodiments above into an extracorporeal setting blood flow circuit in a pre- or post dilution mode of a dialysis filter.

Still another method to reduce, treat and/or prevent oxidative stress and cell activation comprises administering a solution according to the invention in an extracorporeal setting through the membrane by adding the solution to the dialysis fluid.

If human serum albumin is added to the dialysis solution this is added in an amount of 0.2-20 weight-%.

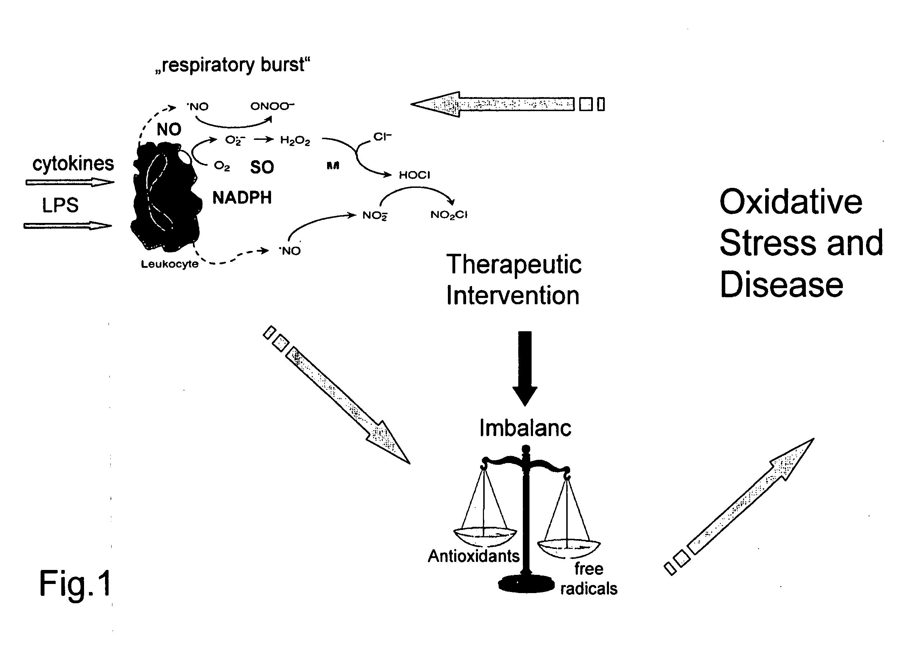

We refer to fig.1. Antioxidants are often referred to as having potentially therapeutic effects in metabolic disturbances in acute and chronic situations like kidney- and liver failure.

The formation of reactive oxygen species is a major component of the host defence mechanism in a human being. Oxidative balance is an integral component of the internal milieu homeostasis resulting from a perfect balance between oxidative and anti-oxidative mechanism. An imbalance of this system is called oxidative stress and results both from impaired radical scavenging system and enhanced reactive oxygen species. Cell activation and oxidative stress is recognised as a general metabolic disturbance leading to severe tissue destruction and malfunction of organs, for example kidney and liver and also the destruction of the vascular system.

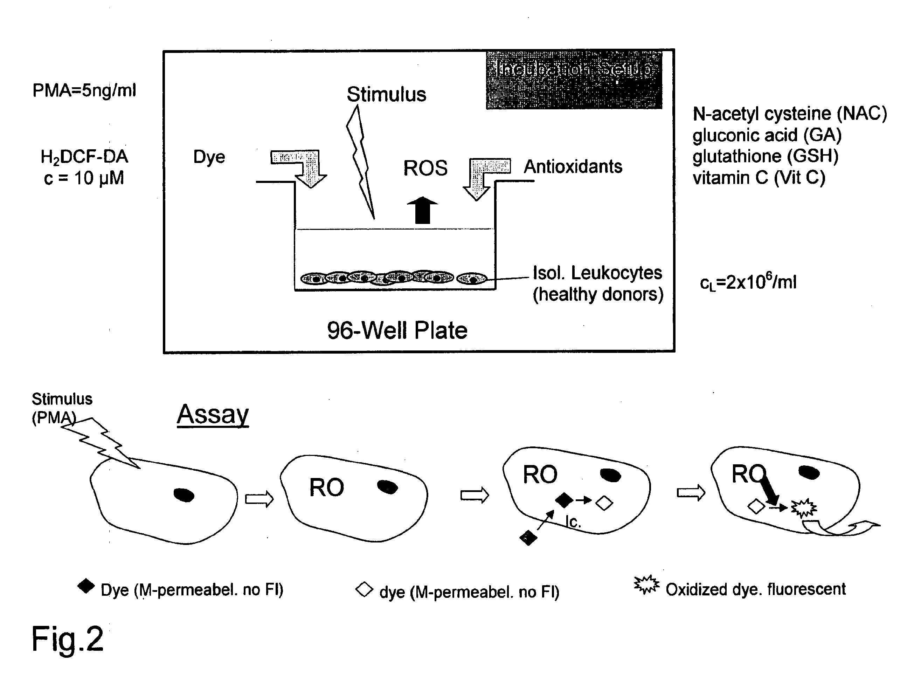

An often discussed therapeutic intervention is the addition of substances with antioxidative properties to balance the oxidative/antioxidative system. In our work we performed a systematic screening of such antioxidants, see fig. 2. The screening was done in a 96-well-plate setup where isolated Leukocytes from healthy donors were pre-incubated with antioxidants for 1 hour.

The chosen substances were N-acetyl-cysteine (NAC), gluconic acid (GA), glutathione (GSH) and vitamin C (Vit C). A fluorescence dye was added as a marker for intracellular free radical formation. Afterwards the cells were stimulated by phorbol-myristate-acetate (PMA) and free radical formation was recorded as fluorescence signal over time.

The dye in this assay is membrane-permeable and non-fluorescent when it is added. Diffusing once into the cell, enzymes convert it to a readily oxidizable fluorescence dye and the intracellular formation of free radicals can be measured.

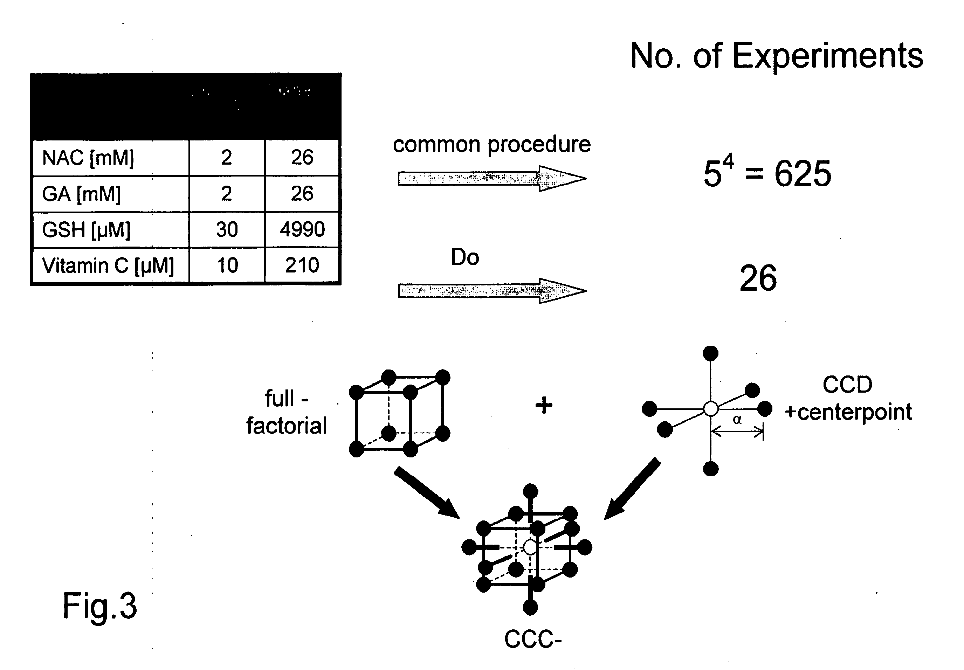

In order to perform a systematic screening of these substances we would have had to carry out a lot of experiments. Due to the lack of blood donors we chose the methodology of design of experiments, see fig.3. Here an experimental plan is generated on statistical basis and a mathematical model can be established. In our case we chose a Centrum Composite Circumscribed - Design (CCC), which is a combination of a full-factorial and a Central Composite - Design (CCD) with a center point. Every single experiment was carried out three times.

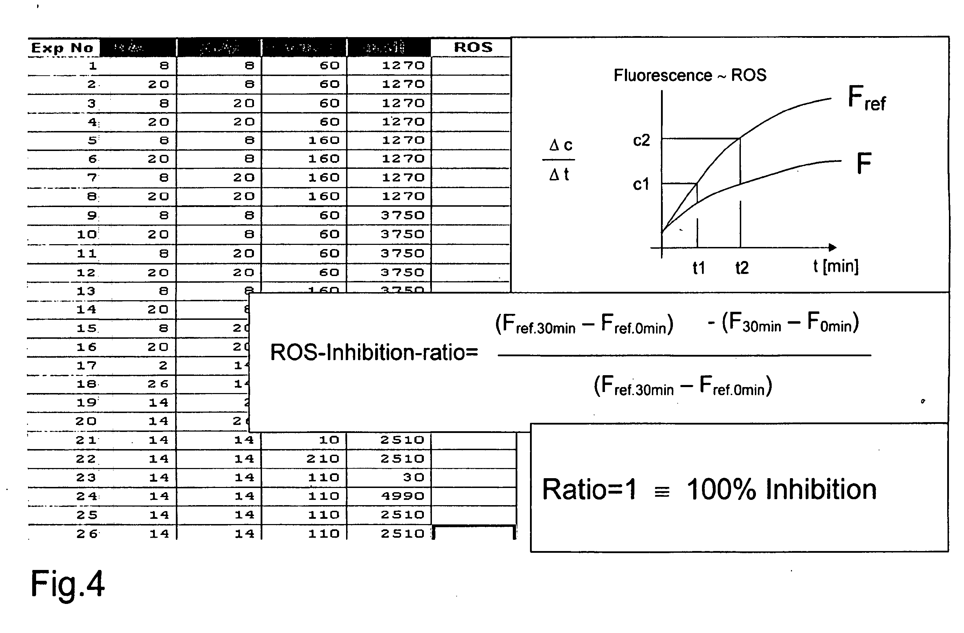

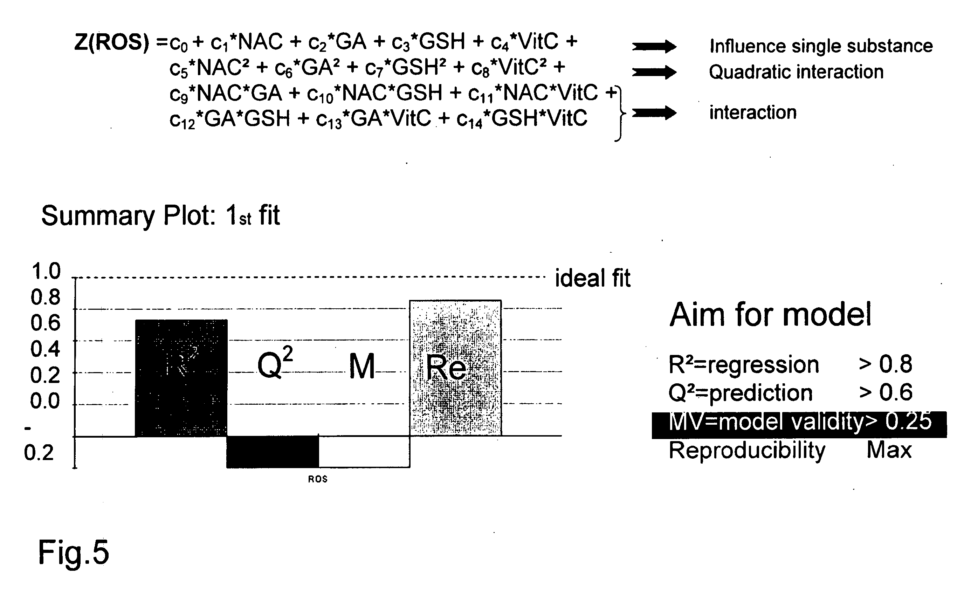

Besides saving many experiments an additional advantage is the consideration of interactions and quadratic influences. The software tool Mode generates the experimental plan where the combinations of the substances vary according to the chosen CCC-Design, see fig.4. Out of the measured fluorescence kinetics a free-radical-inhibition-ratio was calculated and implemented. A ratio of 1 relates to 100% inhibition of free radical formation. The obtained data was fitted by multiple linear regression analysis, see fig.5. The influence of single substances, its quadratic terms and interactions between the substances are considered in the shown equation. The quality of the model fit is defined in the summary plot. R² stands for regression, Q² for prediction quality, MV for model validity and Rep for reproducibility. A first fit to the model does not reach the aim. The model has to be further optimised. To identify a model-weakness a closer look into the model is required.

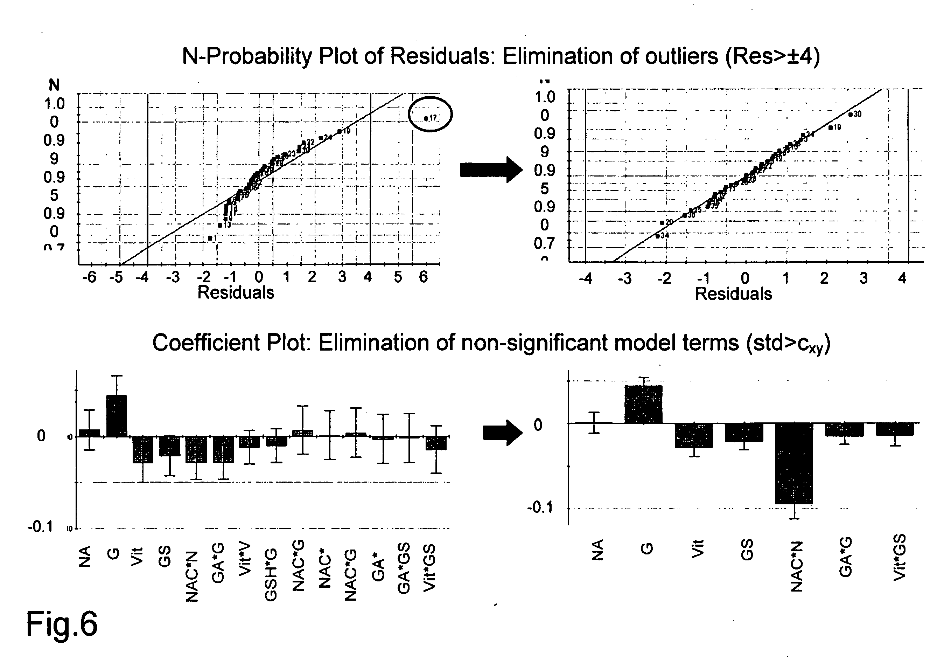

In fig.6 a diagram of the normal distribution of the residuals is shown. It highlights the model fit compared to the measured values. If a value is greater than plus minus three it will be defined as an outlier. Experiment No. 17 is an outlier and will be eliminated. This results in a well distributed N-probability plot of the residuals.

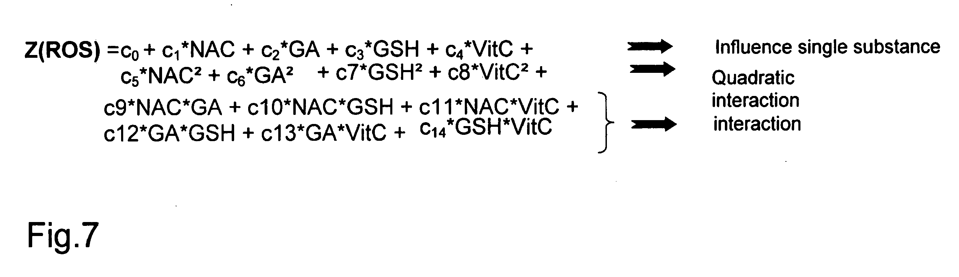

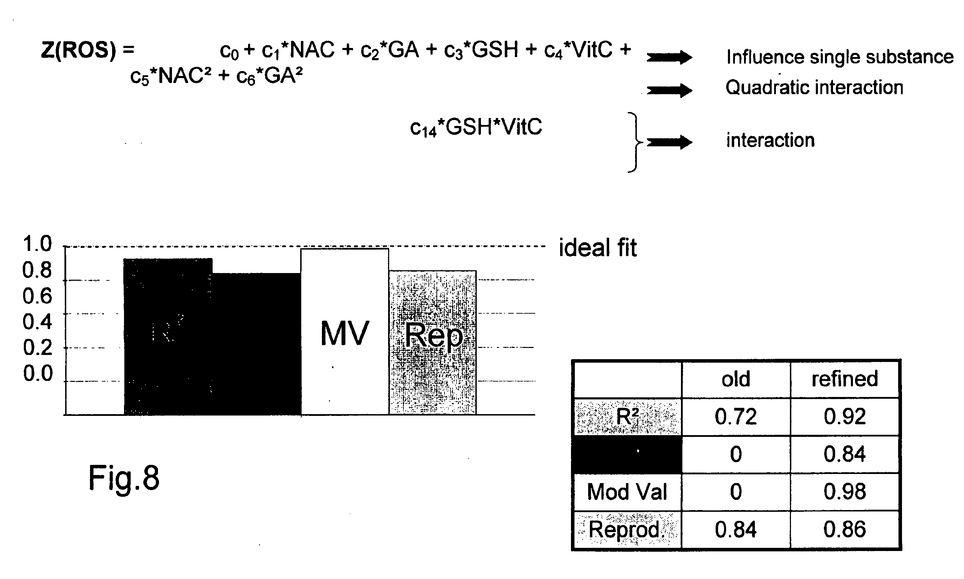

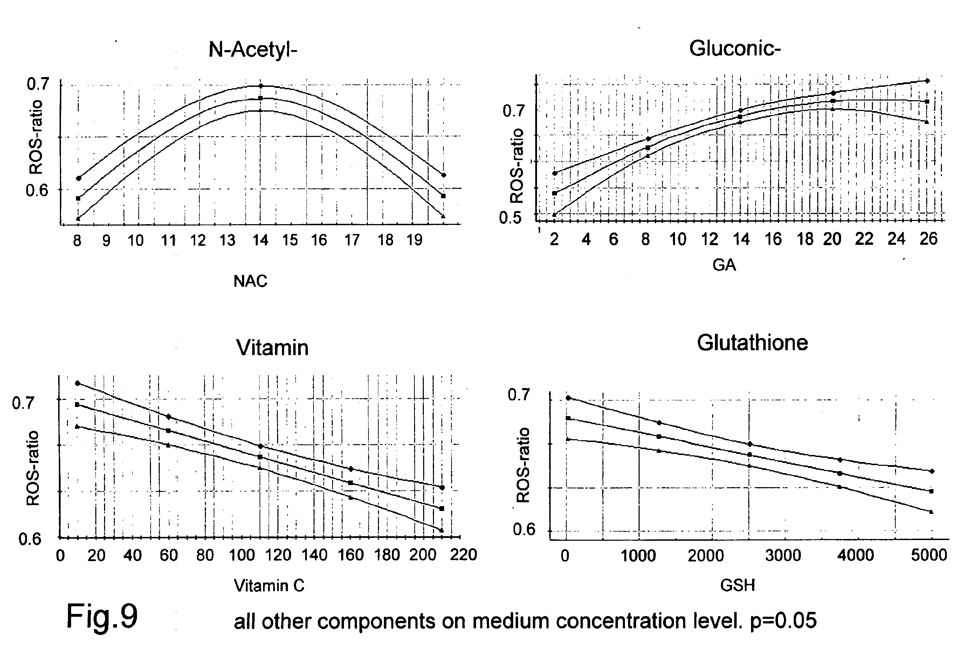

In a second step we had to have a closer look at the coefficient plot. It gives information about non-significant model terms in the mathematical equation. Those are defined as having a higher standard deviation compared to its magnitude. Most of the interaction terms are non-significant and therefore can be eliminated. This results in a simplified coefficient plot with only significant model terms, see fig.7 and 8. These changes have a direct effect on the original equation, which is now simplified. There are only two quadratic interactions and the interaction between glutathione and vitamin C left. Fitting the data again to the new model equation gives a fairly good summary Plot. Compared to the original model all model parameters fulfil the criteria for a sufficient model. We established a significant mathematical model, which is able to predict the outcome on free radical inhibition of different combinations of antioxidants. In the diagrams in fig.9 the influence of the concentration of a single substance in relation to the ROS-Inhibition ratio is shown. In all scenarios the concentrations of the missing substances in the combination are on a medium level and the confidence interval of 0.95 is shown. Remarkable is the quadratic character of N-acetyl-cysteine and gluconic acid, which can be seen in the upper diagrams. Also the negative gradient with increasing concentration for vitamin C and glutathione is shown.

But the most interesting question is: What is the optimal combination in order to maximize the ROS-inhibition?

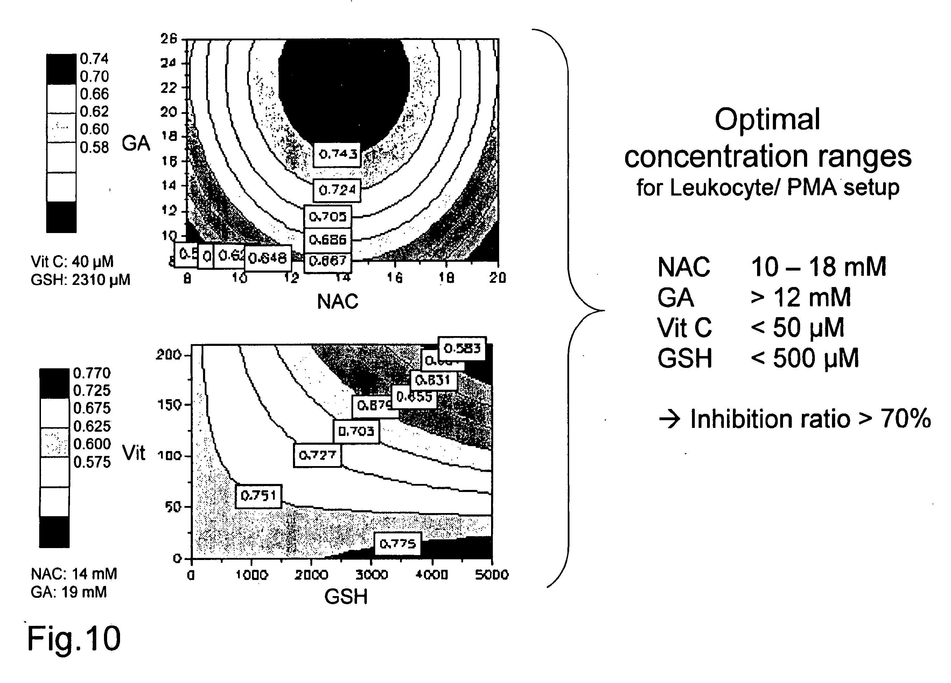

The contour plot together with the optimizer allows to identify the optimal concentration ranges, see figure 10. In the upper diagram NAC and GA are shown, in the diagram below VitC and GSH are shown. The inhibition ratio varies from 0.57 to 0.77 which is equal to 57% to 77% inhibition of free radical formation. Finally the best combination of the tested substances in our experimental test setup results in the shown concentration ranges with an inhibition ratio of greater than 70%. In order to test our model on its robustness against the donor variability, we performed another two independent experiments.

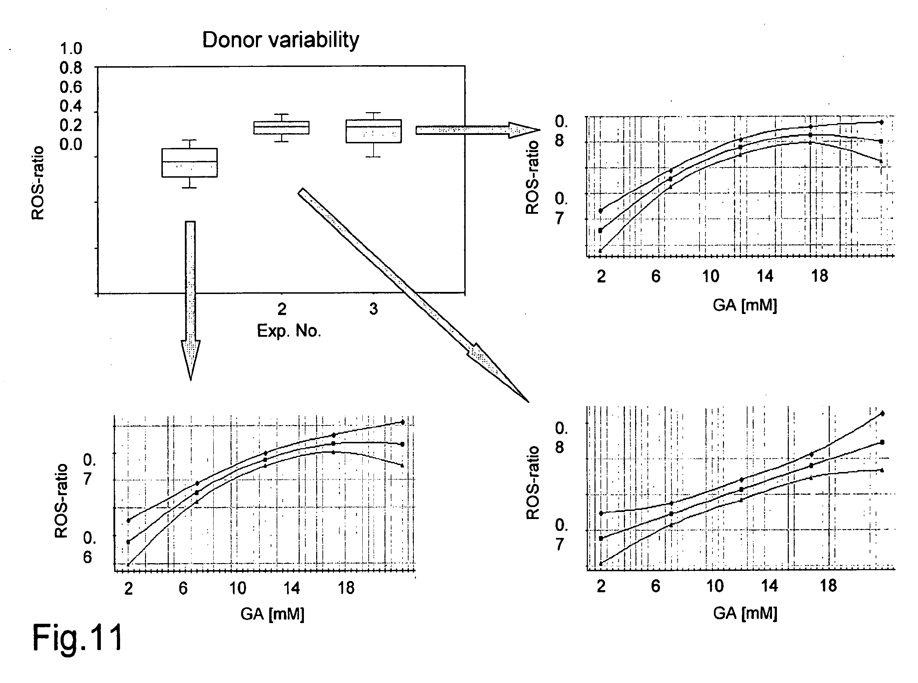

In the diagram in fig. 11 the overall variability and its statistical distribution of each single experiment is shown for all three donors. Fitting the model to these new values we recognised a fairly good model fit, here exemplary shown for gluconic acid.

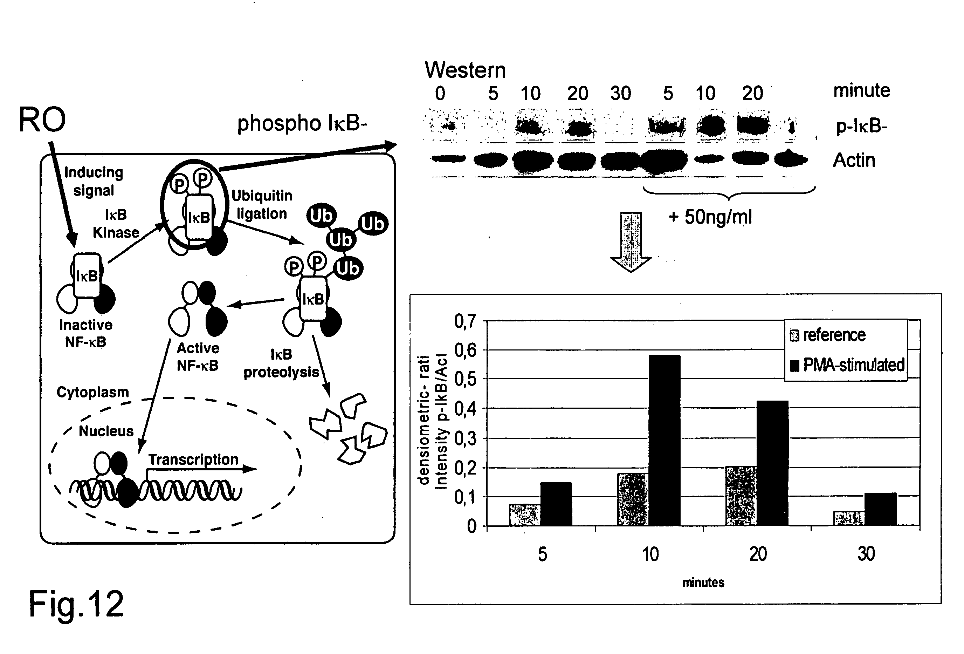

In a next step we want to address the biological effect of the free radicals on the intracellular signalling, see fig.12. NfkappaB plays a central role in immune response and inflammation. Janssen-Heininger YM, Poynter ME, Baeuerie PA., in "Recent advances towards understanding redox mechanisms in the activation of nuclear factor kappaB. " Free Radic Biol Med. 2000 may 1;28(9):1317-27*. Review.* showed that free radicals can directly or synergistically mediate an upregulation of Nf-kB activation. One step of this mechanism is the phosphorylation of ikb, which is the inhibitor of the nfkb subunits in the cytosol. Measuring the grade of phosphorylation of IkB via Western Blot gives information about the activation of the nfkb- pathway. As a summary, we could show that combinations of antioxidants are able to inhibit intracellular ROS- formation.

We used a simple statistical approach and the obtained model allows a better description of biological system. An optimal concentration range could be identified in our test setup and the model robustness against donor variability could be shown. The design has to be refined with respect to timing and stimuli. The model has to be expanded to other read-out parameters like proinflammatory cytokines or activation of nfkb pathway.

As disclosed earlier above, one embodiment of the invention the solution according to the invention further adds an amount of 0,2 - 2 weight-% human serum albumin (HSA) to the blood flow or alternatively the solution further comprises 0.2-20 weight-% HSA.

Experiments have been made to investigate the synergistic effect by combining the different substances. The screening was done in a 96-well-plate setup where isolated Leukocytes from healthy donors were pre-incubated with antioxidants for 1 hour.

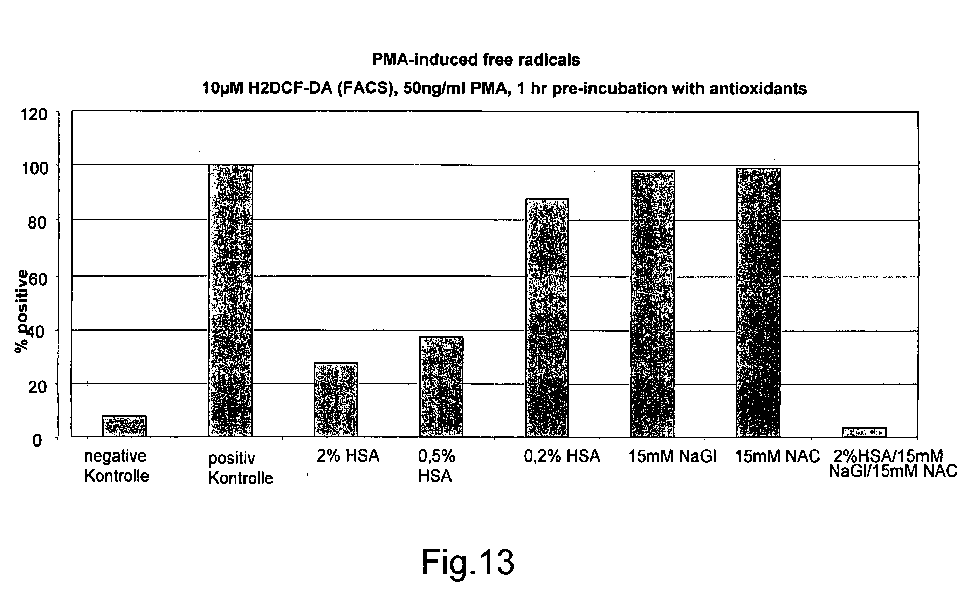

The chosen substances were 2 weight-% HSA, 0.5 weight-% HSA, 0.2% HSA, 15 mM NaGl, 15 mM NAC and 2%HSA together with 15 mM NaGl, and 15 mM NAC. A fluorescence dye was added as a marker for intracellular free radical formation. Afterwards the cells were stimulated by phorbol-myristate-acetate (PMA) and free radical formation was measured by flow cytometry (FACS). The dye in this assay is membrane-permeable and non-fluorescent when it is added. Diffusing once into the cell, enzymes convert it to a readily oxidizable fluorescence dye and the intracellular formation of free radicals can be measured. As is evident from the test results on the fig.13, there is a synergistic effect when combining HSA together with the rest of the antioxidants.

### DESCRIPTION OF THE INVENTION

The proof of principle of a new so far not known concept was done in four steps:

### 1) Inhibition of cell activation / ROS- generation by addition of free radical scavengers

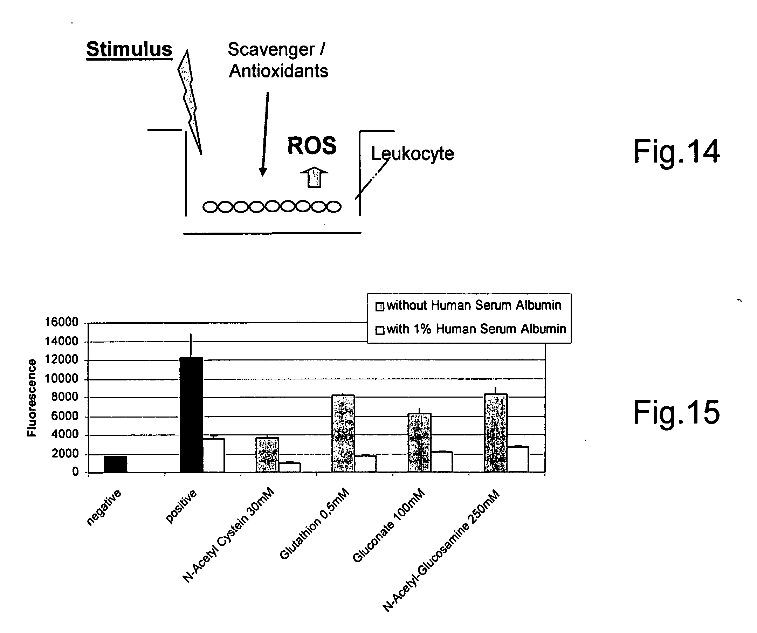

Phorbol-myristate-acetate (PMA) was used as a very effective cell activation agent to induce ROS formation and other cell activation markers, see fig.14. Albumin was used as a macromolecular scavenger as known for high SH content and binding sites for radicals of different chemical species (O₂, H₂O₂, ONOO₂, HOCl). Other macromolecules with SH sites could also be applied.

White blood cells (WBC) were isolated from freshly donated human whole blood. This was done by collecting whole blood from healthy donors on Heparin anticoagulation. Leukocytes were isolated out of the whole blood with the help of an isolation gradient (Polymorphprep). Isolated Leukocytes were diluted in PBS or RPMI 1640 cell culture media and a concentration of 2x10⁶/ml was chosen. The cells were stored at 37° in 5% CO₂. The cells were preincubated with the substances at 37°C for 30-60 minutes. Afterwards the cells were stimulated with 50ng/ml PMA or 30EU/ml LPS. For ROS- measurement a fluorescence dye (dichlorodihydrofluorescein-diacetate, H₂DCF-DA) was added at 10µM final concentration. The cells were incubated with the substance, the fluorescence dye and the activator for 2 hrs and the fluorescence intensity kinetics was measured over time. To compare the result between two substances, the fluorescence intensity at a certain time point, e.g. 30 minutes) was recorded. For comparison the fluorescence at a certain time is shown in the diagram in fig.15.

### Results:

1. N-acetyl-cysteine, glutathione, gluconate and N-acetyl-glucosamine are able to inhibit free radical formation.
2. Mixtures of above-mentioned substances with human serum albumin show an even better effect compared to the single substances, only combinations allow reduced cell activation profile in the range of the negative control.

### Interpretation:

Changing the milieu allows to scavenge the activation signal, most pronounced when combinations are applied. It is surprising that increasing the albumin concentration doesn't enable further signal reduction.

### 2) Inhibition of cell activation / ROS- generation by addition of free radical scavengers via the membrane

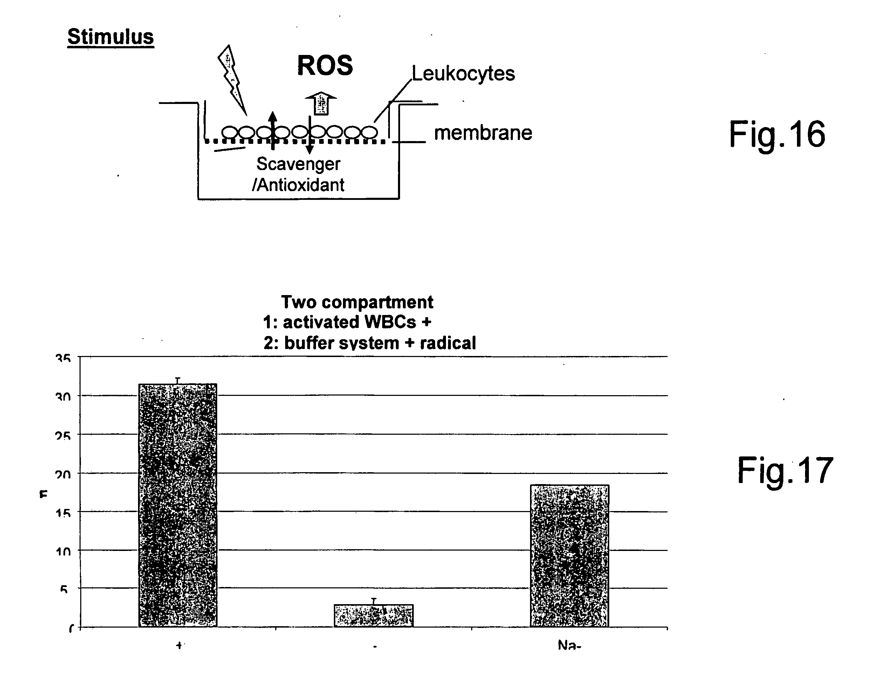

To get a step closer to an extracorporeal treatment system we introduced a membrane (impermeable for albumin (MW 68 000 Da) since low flux type, i.e. cut-off in the range below 10 000 Da) and performed experiments to test whether the interaction of anti-oxidants with the cells across the membrane is able to show an effect, see fig.16.

WBC were isolated from human blood donation. Cells were separated by a membrane from the second compartment which comprises a buffer-system containing a radical scavenger (incubation time 60 mins). Afterwards a fluorescence dye (H₂DCF-DA) was added to the cells and incubated for 25 mins. Finally stimulation of WBCs was performed by PMA and fluorescence was measured over time, see fig.17.
Positive control: with stimulus (2^{nd} compartment PBS- buffer),
Negative control: without stimulus (2^{nd} compartment PBS- buffer)

### Result:

Gluconate is capable to inhibit ( e.g. free radical formation) through the membrane. This indicates that the milieu can be changed in this particular fashion.

### 3) Reduction of ROS during perfusion through minimodule

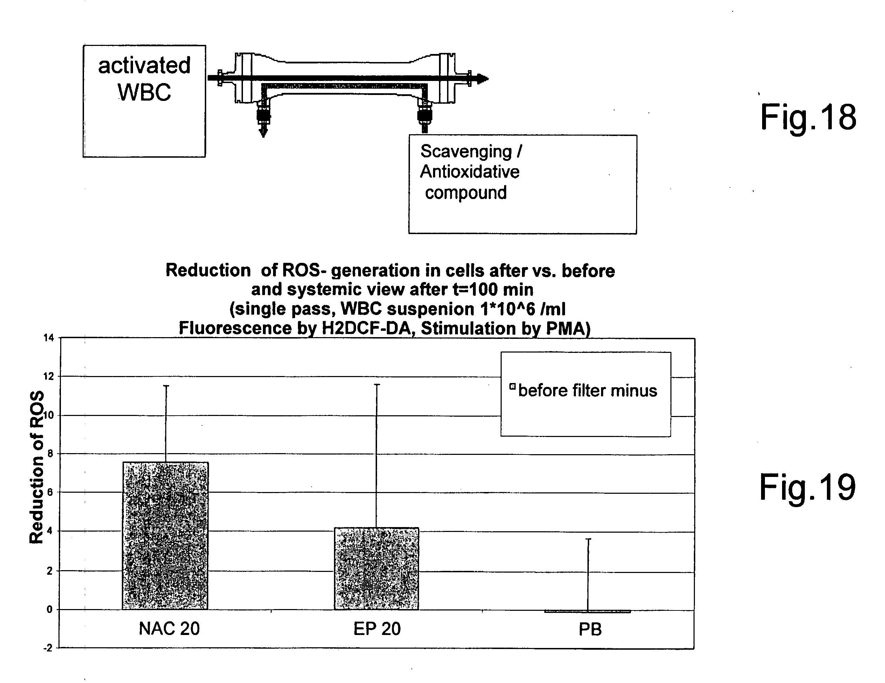

The next question was to apply it in a dynamic system and show that the reaction kinetics are fast enough and enable perfusion / on line application in a refined dialysis or blood treatment system, see fig.18.

WBC were isolated from human blood donation. Cells were stimulated by PMA and after one hour incubation a fluorescence dye (H₂DCF-DA) was added to the cells and incubated for another 25 mins. The cell suspension was pumped single pass through a hollow fiber minimodule. On the dialysate pathway dialysis fluid with or without radical scavenger was pumped in counter current. Fluorescence was measured at the dialyzer inlet and outlet and fluorescence reduction in the filter was calculated. Negative control: PBS- buffer without radical scavenger as dialysis fluid, see fig.19.

### Result:

When NAC or, for comparison, EP(ethyl pyruvate) as examples along the previous experiments are added to the "dialysis" fluid free radicals are reduced on the "blood side" in the dialyzer as well as in the intracellular compartment.

These findings show the applicability in dynamic systems and surprisingly the principle works over a synthetic and a biological membrane barrier. The synthetic barrier is impermeable for albumin.

### 4) Reduction of IL6- expression due to addition of ethyl pyruvate and N-acteyl-cysteine/ Reduction of CD11b adhesion factors

Now the question has been addressed to show whether just radicals / ROS are reduced or whether we could reduce a cell activation signal without being in direct contact with the cellular compartment.

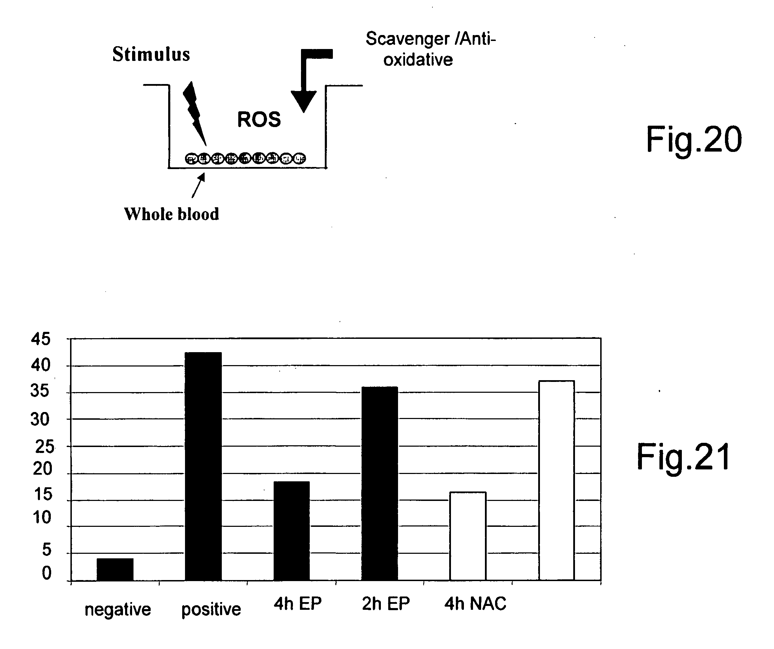

Further we analyzed intracellular IL-6 concentrations - indicator of proinflammatory cell activation - by flow cytometry and by this we were also able to analyze the percentage of ROS-positive / activated cells, see fig.20. Shown are the results for intracellular IL-6. Further data were generated to show:
(1) That use of LPS as primary stimulus instead of PMA (i.e. other cell activation pathway) is inducing similar activation pattern. LPS could stand for a septic / bacterial infection environment instead of a pro-oxidative environment.
(2) That additional and more complete interventions in the pathophysiological sequence through scavengers reduces also the expression of cellular surface markers like CD11b (an indicator of enhanced adhesion to vascular walls).

Whole blood was incubated with LPS; ethyl pyruvate and N-acetyl-cysteine, respectively were added directly or after 2 hrs. Expression of Interleukin 6 was measured after 4 hrs with flow cytometer, see fig.21.

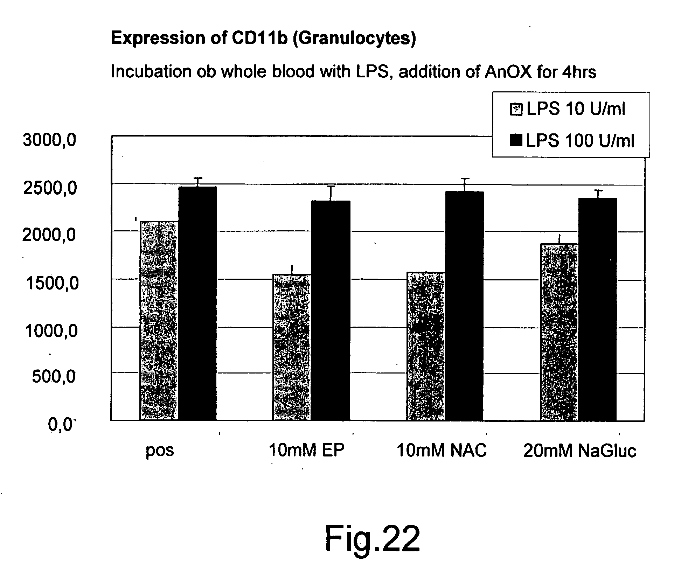

Whole blood was incubated with LPS (10/100U/ml) for 45 minutes. Ethyl pyruvate, N-acetyl-cysteine and gluconate were added to the stimulated system and incubated for 4 hrs. CD11b Expression of granulocytes was measured with flow cytometry, see fig.22.

### Results:

N-acetyl-cysteine and ethyl pyruvate are able to reduce the expression of IL6 and reduce the number of IL-6 positive cells in a time dependent fashion.

N-acetyl-cysteine and ethyl pyruvate and gluconate are able to reduce expression of CD11b adhesion marker.

The procedure invented here is able to interfere in cell activation signalling, i.e. cytokine induction as well as respective gene expression.

By this we disclose for the first time that extracorporeal arrangement can be tailored by specific modification to reduce cell activation, a feature beyond and over the removal of toxins.

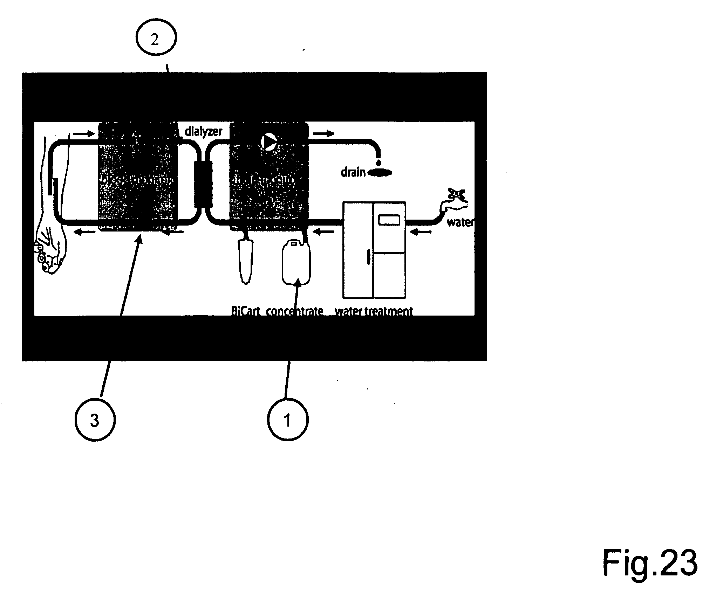

### Possible arrangements for an extracorporeal treatment, see fig.23

| No. | Scavenger source | ROS Scavenging pathway |
|---|---|---|
| 1 | Diffusion and convection from dialysate | Diffusion/convection via the membrane and reaction/scavanging in the dialyzer or just pass the membrane (albumin) |
| 2 | Pre- Infusion | Reaction with ROS in dialyzer and elimination of byproducts and excess Scavenger through the membrane |
| 3 | Post- Infusion | Scavenging in the body (systemic pathway), elimination of excess scavenger and by-products in the dialyzer |

As described before, the application of a solution according to the invention is performed by infusion to the patient in an extracorporeal circuit or by adding it to the dialysis fluid.

The relationship between the applied dosage and the achieved plasma level in the patient is generally descrbed by the parmacokinetics of a substance (pk). Accordingly, studies on the pharmacokinetics of a substance are dedicated to the determination of the fate of such substance administered externally to a living organism.

In order to understand how a solution according to the invention has to be composed, it is important to take into account the pharmacokinetics of the substances involved. It is understood that the determination of the plasma level concentration of any of the compounds described in connection with a solution according to the invention is a process known to a person with skill in the art. Further, it is known to a person with skill in the art how to calculate the concentration of a compound which has to be administered to a person based on the desired plasma level concentration and the height/weight of said person.

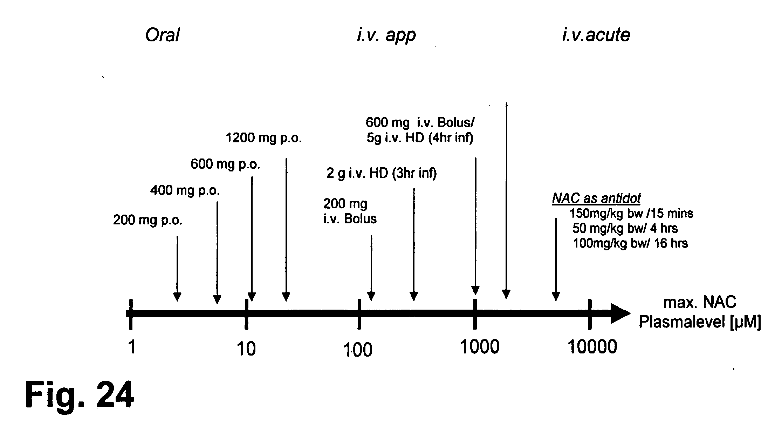

By way of example, fig. 24 depicts the pk for the oral and intravenous application of N-acetyl-cysteine. "o.p." refers to the oral administration of the substance, "i.v." to the intravenous application. "HD" means hemodialysis, "bw" means body wight. Oral application results in low plasma levels o NAC due to poor bioavailability (3-30 µM). Further increasing the plasma level through oral application would require tremendous amounts of drug which would be less biocompatible, i.e. side effects would no longer be acceptable. In contrast, intravenous application results in higher plasma levels depending on the dosage and the application regime. Rather high plasma levels are achieved and well tolerated with the application as an antidote in acute situations. Therefore, the afore mentioned higher concentrations of the compounds in the solutions according to the invention are applicable in situations with an acute need for treating oxidative stress and cell activation, while the lower concentrations or preferred ranges will rather be applicable for a long-term or preventive treatment or prevention of oxidative stress and cell activation.

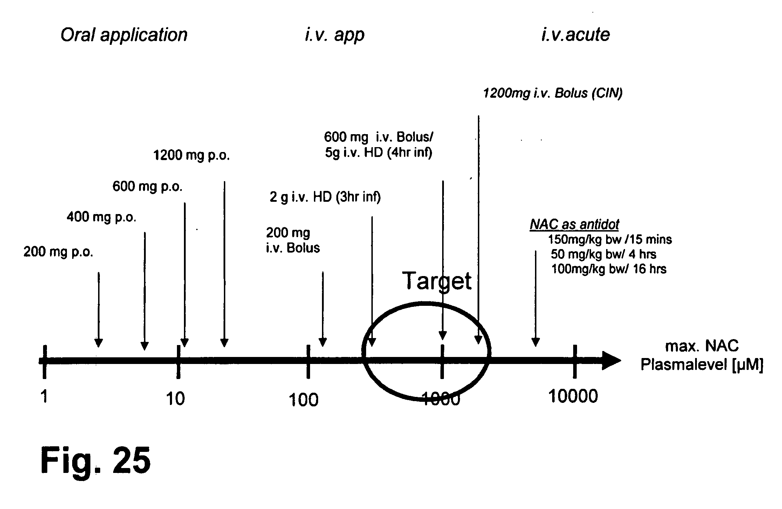

By way of example, fig. 25 further indicates the desirable concentrations of NAC within the blood flow, i.e. the plasma levels which should preferably achieved according to the invention. First beneficial effects could be shown with plasma levels as low as 12 µM of acetylcysteine. Higher plasma levels due to i.v. application in the range of 100 µM to 2 mM are expected to be superior compared to lower levels. Still higher levels of NAC would be applicable but depending on the disease adverse effects have to be considered.

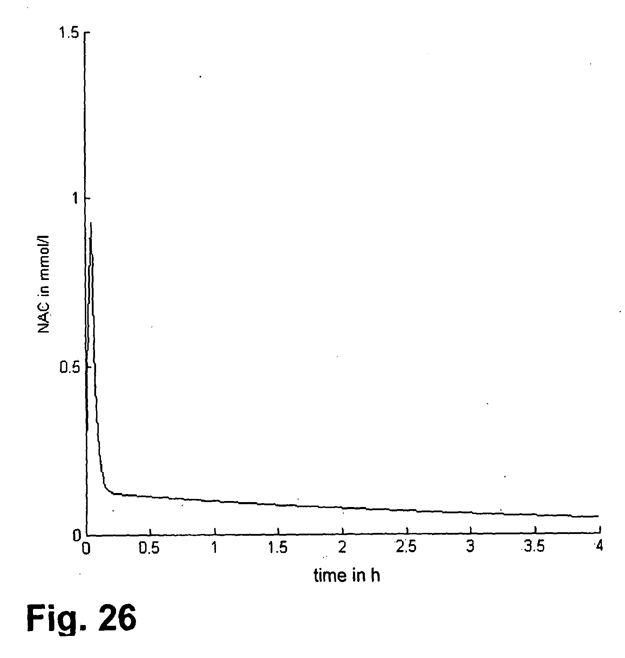

The concentration of the respective components of a solution according to the invention can be further controlled by the infusion regime. Any of the solutions according to the invention could be administered to an animal or human being by bolus injection. A bolus injection is the injection of a drug (or drugs) in a high quantity (called a bolus) at once or in a short time, the opposite of gradual administration (as in intravenous infusion). The expected pharmacokinetics is, by way of example, depicted for NAC in fig.26. The example shows a 600 mg bolus injection over 3 minutes for a haemodialysis patient. A high initial concentration of N-acetyl-cysteine is achieved with this approach helping to cope with the stimulation/activation soon after application. A disadvantage, however, is the fast decreasing concentration resulting in a low bioavailability once the injection is stopped.

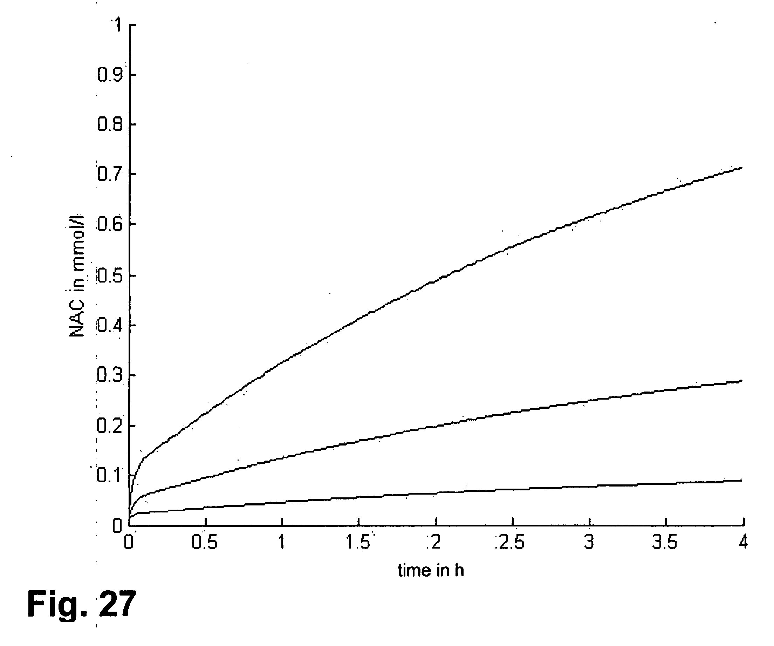

The solutions according to the invention may be administered by means of a continuous infusion regime with slowly increasing plasma levels. The expected pharmacokinetics is depicted, by way of example,in fig.27. The diagram shows continous infusions of 5g (top), 2g (middle) and 0,6 g (bottom line) during an extracorporeal treatment (haemodialysis). Due to the continuous infusion a steadyly increasing concentration of NAC is achieved and thus a constant good bioavailability. However, high initial N-acetyl-cysteine levels cannot be achieved due to distribution, metabolism and elimination effects. While this may be acceptable in some cases when the treatment of acute oxidative stress and cell activation is not indicated, this may be a disadvantage in other cases.

Therefore, a high initial NAC plasma level concentration and a higher bioavailability along the treatment can be achieved by combining the aforementioned ways of administering a solution according to the invention, i.e. by administering a first bolus injection before or at the time of the beginning of a continuous infusion.

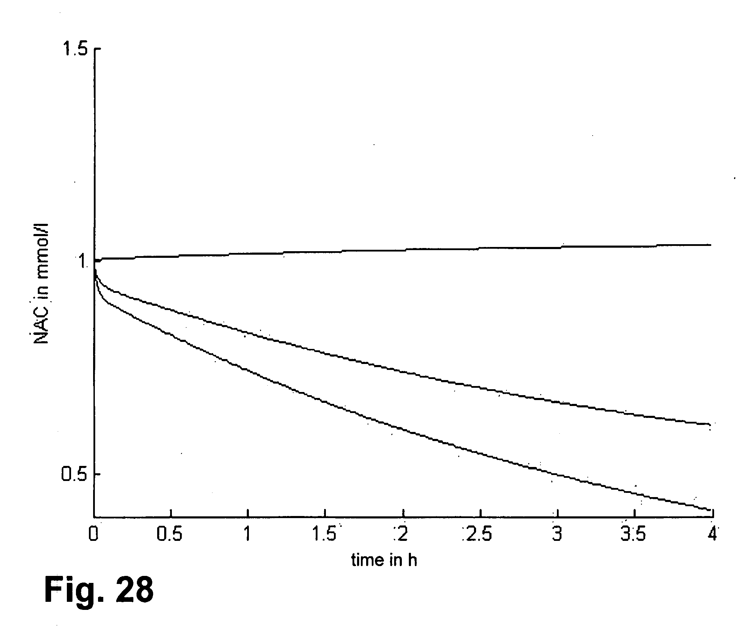

By way of example, the time course of NAC plasma level concentration is depicted in fig. 28 for various concentrations used for continuous injection (5g, 2g and 0.6g of NAC).

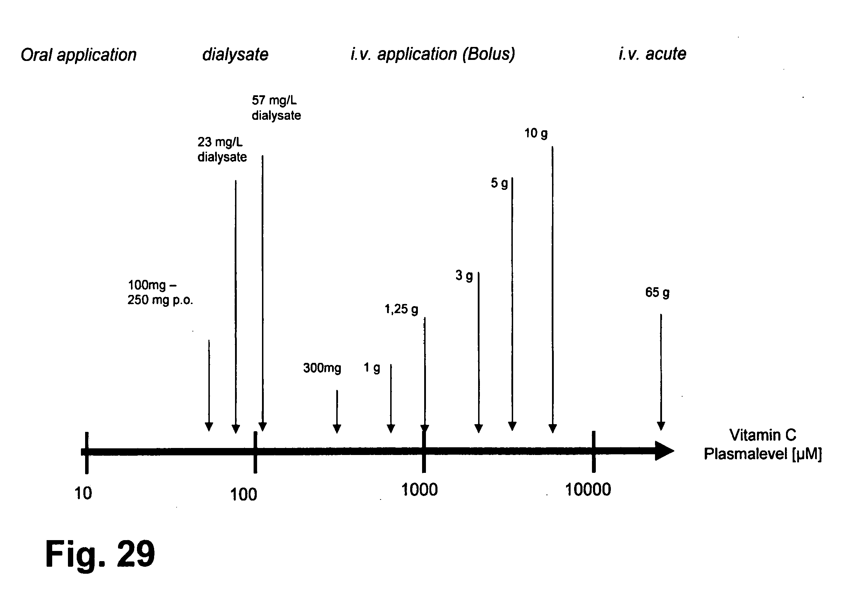

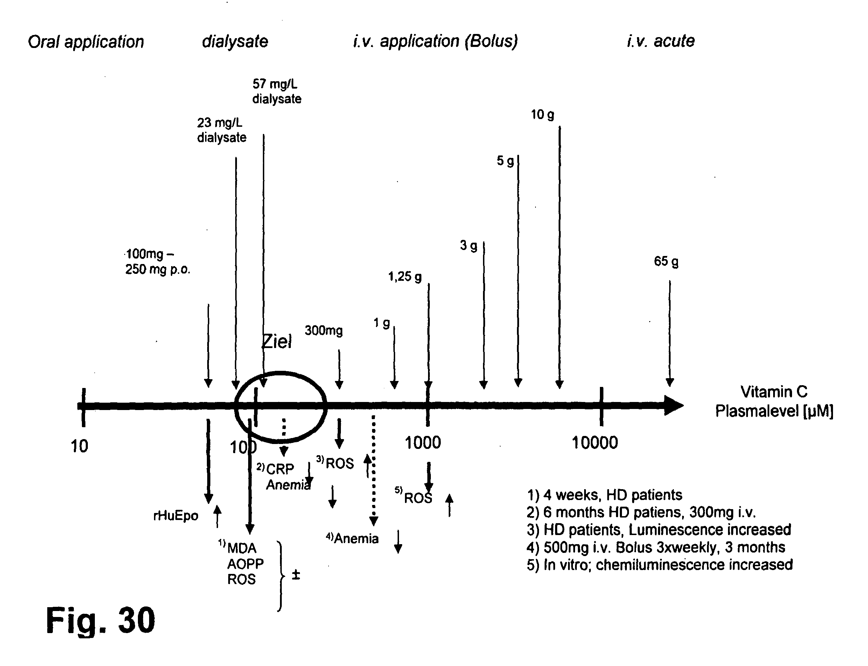

The considerations taken above for the pharmacokinetics of NAC can of course also be transferred, for example, to vitamin C. Vitamin C may be applied over a wide concentration range. Intravenous applications are superior compared to oral applications regarding the bioavailability of the substance. This can again be seen from fig. 29. However, very low concentrations of vitamin C in the plasma are not effective, while very high concentrations are reported to have adverse effects, depending on the disease. The best concentration range in the blood as determined according to the invention would be from 0.01-0.21 mM, preferably from 0.05-0.2 mM. This is again depicted in fig. 30.

The same consideration of course also apply to the remaining components of any of the solutions according to the present invention, i.e. gluconic acid and glutathione.

In a further aspect, the preferred point in time for a treatment in order to prevent or treat oxidative stress and cell activation was investigated. Therefore, the ROS-inhibition was tested in two different applications:
1. Pre-incubation (activation of cells after incubation with a substance or combination of substances according to the invention)
2. Post-incubation (addition of a substance or a combination of substances according to the invention after cell activation)

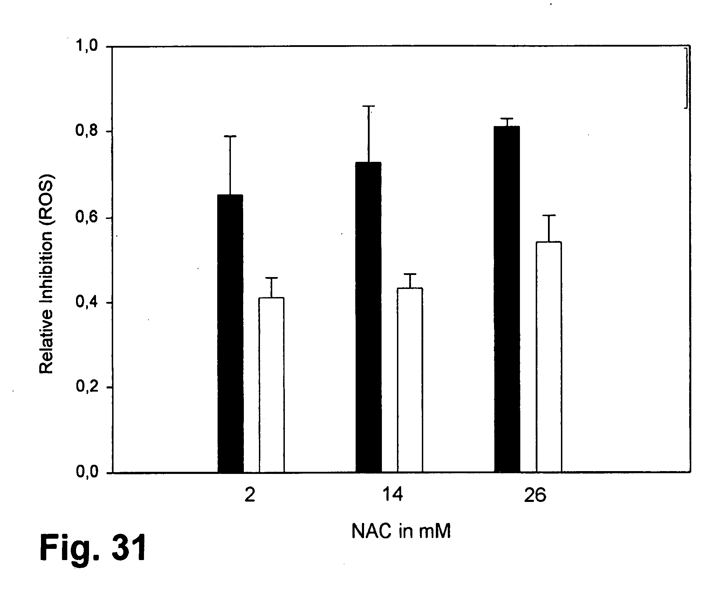

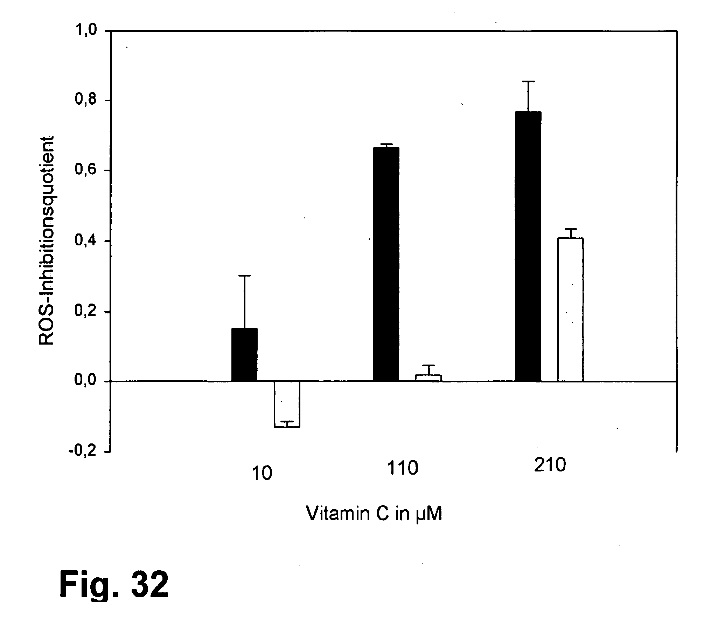

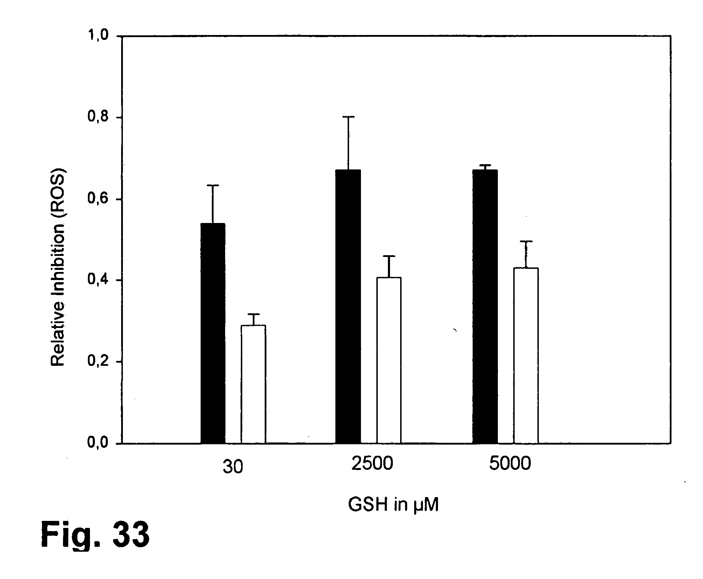

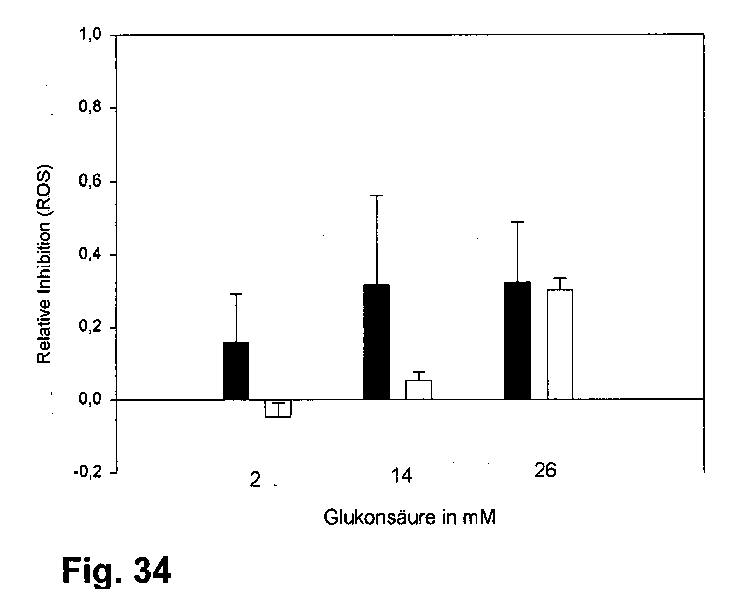

All substances or combinations thereof were tested in the same concentration ranges as in the screening of the combinations as described before. The results for the single substances are depicted in figures 31 to 34. In said figures, light grey columns show the results after pre-stimulation, while dark columns show the results after pre-incubation. As can be seen from the figures, in each case pre-incubation with a substance according to the invention is beneficial compared to pre-stimulation regarding the inhibition of ROS. The ROS inhibition is different for all substances tested: NAC (60-80%), glutathione (55-65%), vitamin C (20-80%) and gluconic acid (15-30%).

Therefore, a method of preventing or reducing oxidative stress and cell activation comprises infusing anyone of the solutions according to invention into the blood of a patient in need thereof preferably before the expected onset of oxidative stress and cell activation, preferably 50 to 60 minutes before. The onset of oxidative stress and cell activation can, for example, be expected in cases of extracorporeal treatments, such as, for example, hemodialysis, even though a patient being in need of such treatment may already suffer from oxidative stress and cell activation before such treatment. Therefore, it may be desirable to treat any such patient also during said extracorporeal treatments or in between such treatments.

## Claims

1. A solution for use in reducing, treating and/or preventing oxidative stress and cell activation, comprising N-acetyl-cysteine in a concentration which gives rise to a concentration of N-acetyl-cysteine within a blood flow of 0.1-18 mM, and further comprising gluconic acid in a concentration which gives rise to a gluconic acid concentration within a blood flow of 0.4-26 mM, if the solution is administered to a human or animal body.

2. A solution for use according to claim 1, wherein the solution gives rise to a concentration of N-acetyl-cysteine within a blood flow of 0.1-10 mM.

3. A solution for use according to claim 1 or 2, which further comprises vitamin C in a concentration which gives rise to a vitamin C concentration within a blood flow of 0.01-0.21 mM.

4. A solution for use according to claim 3, which comprises vitamin C in a concentration which gives rise to a vitamin C concentration within a blood flow of 0.05-0.02 mM.

5. A solution for use according to any one of claims 1 to 4, which comprises gluconic acid in a concentration which gives rise to a gluconic acid concentration within a blood flow of 0.4-12 mM.

6. A solution for use according to any one of claims 1 to 5, which further comprises glutathione in a concentration which gives rise to a concentration of glutathione within a blood flow of 0.01-5 mM glutathione.

7. A solution for use according to claim 6, which comprises glutathione in a concentration which gives rise to a concentration of glutathione within a blood flow of 0.01-0.5 mM.

8. A solution for use according to anyone of claims 1 to 7, wherein the solution gives rise to an additional amount of 0.2-2 weight-% human serum albumin in the blood flow.

9. A solution for use according to anyone of claims 1 to 7, wherein the solution further comprises 0.2-20 weight-% human serum albumin.

## Patentansprüche

1. Lösung zur Verwendung für die Reduktion, Behandlung und/oder Vermeidung von oxidativem Stress und Zellaktivierung, umfassend N-Acetylcystein in einer Konzentration, die eine Erhöhung der Konzentration von N-Acetylcystein im Blutstrom um 0.1-18 mM nach sich zieht, und weiterhin umfassend Gluconsäure in einer Konzentration, die eine Erhöhung der Konzentration von Gluconsäure im Blutstrom um 0.4-26 mM nach sich zieht, wenn die Lösung dem menschlichen oder tierischen Körper zugeführt wird.

2. Lösung zur Verwendung gemäß Anspruch 1, worin die Lösung eine Erhöhung der Konzentration von N-Acetylcystein im Blutstrom um 0.1-10 mM nach sich zieht.

3. Lösung zur Verwendung gemäß Anspruch 1 oder 2, worin die Lösung zusätzlich Vitamin C in einer Konzentration enthält, die eine Erhöhung der Vitamin C Konzentration im Blutstrom um 0.01-0.21 mM nach sich zieht.

4. Lösung zur Verwendung gemäß Anspruch 3, die Vitamin C in einer Konzentration umfasst, die eine Erhöhung der Vitamin C Konzentration im Blutstrom um 0.05-0.02 mM nach sich zieht.

5. Lösung zur Verwendung gemäß einem der Ansprüche 1 bis 4, die Gluconsäure in einer Konzentration umfasst, die eine Erhöhung der Gluconsäure Konzentration im Blutstrom um 0.4-12 mM nach sich zieht.

6. Lösung zur Verwendung gemäß einem der Ansprüche 1 bis 5, die zusätzlich Glutathion in einer Konzentration umfasst, die zu einer Erhöhung der Glutathion Konzentration im Blutstrom um 0.01-5 mM Glutathion nach sich zieht.

7. Lösung zur Verwendung gemäß Anspruch 6, die Glutathion in einer Konzentration umfasst, die eine Erhöhung der Glutathion Konzentration im Blutstrom um 0.01-0.5 mM nach sich zieht.

8. Lösung zur Verwendung gemäß einem der Ansprüche 1 bis 7, worin die Lösung eine Erhöhung der Menge von humanem Serumalbumin von zusätzlich 0.2-2 Gew.-% im Blutstrom nach sich zieht.

9. Lösung zur Verwendung gemäß einem der Ansprüche 1 bis 7, worin die Lösung zusätzlich 0.2-20 Gew.-% humanes Serumalbumin umfasst.

## Revendications

1. Solution destinée à être utilisée pour réduire, traiter et/ou prévenir des contraintes par oxydation et l'activation des cellules, comprenant de la N-acétyl-cystéine dans une concentration qui entraîne une concentration de N-acétyl-cystéine dans un flux sanguin de 0,1 à 18 mM, et comprenant en outre de l'acide gluconique dans une concentration qui entraîne une concentration d'acide gluconique dans un flux sanguin de 0,4 à 26 mM, si la solution est administrée à un corps humain ou animal.

2. Solution destinée à être utilisée selon la revendication 1, dans laquelle la solution entraîne une concentration de N-acétyl-cystéine dans un flux sanguin de 0,1 à 10 mM.

3. Solution destinée à être utilisée selon la revendication 1 ou 2, comprenant en outre de la vitamine C dans une concentration qui entraîne une concentration de vitamine C dans un flux sanguin de 0,01 à 0,21 mM.

4. Solution destinée à être utilisée selon la revendication 3, qui comprend de la vitamine C dans une concentration qui entraîne une concentration en vitamine C dans un flux sanguin de 0,05 à 0,02 mM.

5. Solution destinée à être utilisée selon l'une quelconque des revendications 1 à 4, qui comprend de l'acide gluconique dans une concentration qui entraîne une concentration d'acide gluconique dans un flux sanguin de 0,4 à 12 mM.

6. Solution destinée à être utilisée selon l'une quelconque des revendications 1 à 5, qui comprend en outre du glutathion dans une concentration qui entraîne une concentration de glutathion dans un flux sanguin de 0,01 à 5 mM de glutathion.

7. Solution destinée à être utilisée selon la revendication 6, qui comprend du glutathion dans une concentration qui entraîne une concentration de glutathion dans un flux sanguin de 0,01 à 0,5 mM.

8. Solution destinée à être utilisée selon l'une quelconque des revendications 1 à 7, dans laquelle la solution entraîne une quantité additionnelle de 0,2 à 2 % en poids d'albumine de sérum humain dans le flux sanguin.

9. Solution destinée à être utilisée selon l'une quelconque des revendications 1 à 7, dans laquelle la solution comprend en outre de 0,2 à 20 % en poids d'albumine de sérum humain.
